(19) Europäisches Patentamt
European Patent Office
Office européen des brevets

(11) EP 2 638 857 A1

(12) EUROPÄISCHE PATENTANMELDUNG

(43) Veröffentlichungstag:
18.09.2013 Patentblatt 2013/38

(51) Int Cl.:
A61B 6/00 (2006.01)

(21) Anmeldenummer: 12178674.3

(22) Anmeldetag: 31.07.2012

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(30) Priorität: 11.03.2012 RU 2012108684

(71) Anmelder:
• Space Research Institute (IKI)
Moscow 117997 (RU)
• Russian Scientific Center
Moscow 117997 (RU)

(72) Erfinder:
• Gorshkov, Viacheslav
12147 Moscow (RU)
• Nazirov, Ravil
111672 Moscow (RU)
• Rodin, Viacheslav
119526 Moscow (RU)
• Rozhkova, Nadezhda
117292 Moscow (RU)
• Prokopenko, Sergey
117437 Moscow (RU)

(74) Vertreter: Jeck, Anton
Jeck - Fleck - Herrmann
Patentanwälte
Klingengasse 2/1
71665 Vaihingen/Enz (DE)

Bemerkungen:
Ein Antrag gemäss Regel 139 EPÜ auf Berichtigung der Zeichnungen liegt vor. Über diesen Antrag wird im Laufe des Verfahrens vor der Prüfungsabteilung eine Entscheidung getroffen (Richtlinien für die Prüfung im EPA, A-V, 3.).

(54) **Verfahren zur Zwei-Energien-Divisions-Differenz-Mammographie**

(57) Die Erfindung betrifft ein Verfahren zur Zwei-Energien-Divisions-Differenz- Mammographie. Um die Mikrokalzifikate als Vorläufer eines onkologischen Tumors in der Brustdrüse früher erkennen zu können, sieht die Erfindung vor, dass ein Vergleichsmuster mit bekannten Dichte-, Dicke- und effektiven Atomnummernverteilungen neben der Milchdrüse angeordnet, dass anhand des Vergleichsmusters die Parameter des Zusammenhanges zwischen der Atomnummer und der Differenz bzw. dem Verhältnis der Logarithmen der Anzahl von Fotonen, die die Milchdrüse ohne Zusammenwirken bei zwei unterschiedlichen Strahlungsenergien durchströmen, bestimmt werden, und dass anhand dieses Zusammenhanges die Verteilung der Atomnummern in der Brustdrüse visuell dargestellt wird.

Fig.1

EP 2 638 857 A1

**Beschreibung**

Die Erfindung betrifft ein Verfahren zur Zwei- Energien- Divisions- Differenz- Mammographie nach dem Oberbegriff des Anspruchs 1.

Die Erfindung ist in der Medizin und zwar bei Verfahren zur Diagnose von gut- und bösartigen Erkrankungen der Milchdrüse einsetzbar.

Die Vorläufer eines onkologischen Tumors in der Brustdrüse sind Mikrokalzifikate. Diese haben eine beachtlich größere effektive Atomnummer (Z = 12-14) gegenüber der effektiven Atomnummer eines gesunden Gewebes (Z = 6,5-7,5). Das Vorhandensein von Mikrokalzifikaten ist im Grunde genommen eine ausreichende Voraussetzung für die Bildung eines onkologischen Tumors. Besonders gefährlich sind dabei Mikrokalzifikate, deren Größe unter 200 $\mu$m liegt, denn sie werden durch keine Mittel bei einer röntgenologischen Brustdrüsendarstellung erkannt.

Ein Krebsgeschwulst hat auch eine erhöhte effektive Atomnummer. Das hängt mit einer verschiedenen Kohlenstoff- und Sauerstoffverteilung zusammen (Antoniassi M., Conceição A.L.C. Study of effective atomic number of breast tissues determined using the elastic to inelastic scattering ratio // Nuclear Instruments and Methods in Physics Research Section A: Accelerators, Spectrometers, Detectors and Associated Equipment. 2011. V. 652, No. 1. P. 739—743.).

Ein Verfahren zur Zwei- Energien- Divisions- Differenz- Mammographie ermöglicht, Mikrokalzifikate zuverlässiger und in früheren Krankheitsstadien zu erkennen und eine onkologische Neubildung schärfer darzustellen als es bei den konventionellen Diagnoseverfahren der Fall ist.

Das aussagereichste Diagnoseverfahren bei frühen untastbaren Krebsformen ist die Röntgenscreening-Mammographie. Diesem Verfahren liegt der Effekt zugrunde, dass der Röntgenabsorptionsgrad bei den verschiedenen Geweben unterschiedlich ist. Es handelt sich also um die Visualisierung der Mengenverteilung der Fotonen, die die Milchdrüse ohne jegliche Zusammenwirkung durchströmt haben:

$$N = N_0 e^{-\int_0^d \mu(E,Z,x)\rho(x)dx} \tag{1}$$

wobei

$N_0$    - die Ausgangsfotonenzahl,

$\mu(E, Z, x)$    - die Massenkoeffizientverteilung des Totalabsorptionskoeffizienten über der Strahllinie (der Massenabsorptionskoeffizient ist dabei von der Energie des Ausgangsfotons und von der effektiven Atomnummer des Milchdrüseabschnittes abhängig) ,

$\rho(x)$    - Dichteverteilung entlang des Strahlungsvektors und

$d$    - Milchdrüsendicke entlang des Strahlungsvektors ist.

Der Massenabsorptionskoeffizient ist mit der effektiven Atomnummer (innerhalb seines engen Veränderungsbereiches) proportional.

Somit ist das konventionelle Röntgen-Mammogramm die Darstellung der nichtlinearen Verteilung des Produktes von Dicke, Dichte und der effektiven Atomnummer in der Brustdrüse.

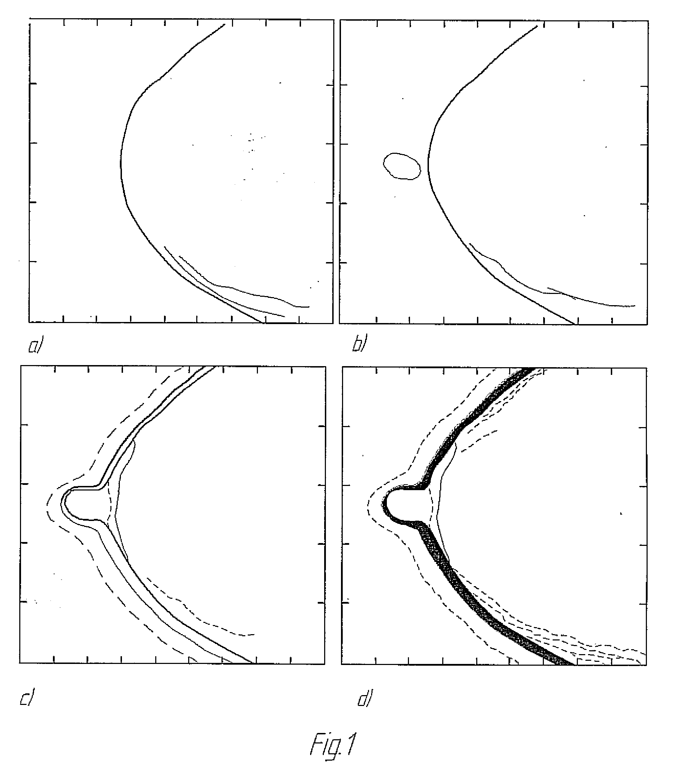

Die Fig. 1a zeigt ein Beispiel für ein konventionelles Mammogramm der Milchdrüse mit Mikrokalzifikaten.

Die Dichtestreuung in der Brustdrüse (Gänge, Gefäße, gutartige Bildungen usw.) deckt die eventuell vorhandenen kleinen Mikrokalzifikate auf den Mammogrammen häufig ab. Trotz der benutzten Kollimatoren erfassen die Detektoren doch die Röntgen-Streustrahlung. Deswegen wird die Milchdrüse auf den Mammogrammen nicht scharf genug abgebildet. Das erschwert wiederum die Erkennung von kleinen Mikrokalzifikaten noch mehr. Nur über 200 $\mu$m große Mikrokalzifikate werden in der Brustdrüse sicher identifiziert. Kleinere Mikrokalzifikate sind nur noch auf homogenen künstlichen Proben (Phantomen) erkennbar.

Um die Empfindlichkeit von Mammogramm gegenüber der Verteilung der effektiven Atomnummer zu steigern, wird in der Praxis weltweit das Verfahren zur Zwei- Energien- Differenz- Mammographie (Zwei- Energien- Subtraktionsmammographie) angewendet. Es ist durch zahlreiche Patente geschützt (Dual energy rapid switching imaging system, US Patent 4541106, 1985; Dual- energy system for quantitative radiographic imaging, US Patent 5150394, 1992; Dual energy x- ray imaging system and method for radiography and mammography, US Patent 6683934, 2004) .

Das Verfahren ist besonders eingehend in folgender Druckschrift beschrieben: Lewin, J. M., Isaacs, P. K., Vance, V., Larke, F. J.: Dual-energy contrast-enhanced digital subtraction mammography: feasibility. Radiology, Volume 229, Number 1, 261-268, (2003).

Nach diesem Verfahren werden zwei Mammogramme mit zwei verschiedenen Energien der Ausgangsstrahlung erzeugt. Danach erfolgt ihre Logarithmierung und Subtraktion (Differenzrechnung):

$$\alpha = \ln \frac{N_0^L}{N^L} - \ln \frac{N_0^H}{N^H} = \rho d(\mu_L - \mu_H) = \rho d(k_\alpha Z + a_\alpha)$$

$$(2)$$

wobei

L, H        Indizes, die dem unteren und den oberen Energiewert entsprechen, und

$k_\alpha, a_\alpha$        Linearisierungskoeffizienten sind.

Damit stellt die Zwei- Energien- Differenz- Mammographie die Visualisierung der linearen Verteilung des Produktes der effektiven Atomnummer und der Dichte dar.

Fig. 1b zeigt ein Beispiel für das Differenzmammogramm für die gleiche Milchdrüse.

Das Zwei-Energien-Differenzmammogramm ist beachtlich schärfer (weil die Streustrahlung unterdrückt wird). Jedoch ist die Darstellung von einzelnen Stellen in der Milchdrüse sowohl von der effektiven Atomnummer als auch von ihrer Dichte und Dicke abhängig. Das erschwert ebenfalls die Erkennung von winzigen Mikrokalzifikaten (es sind nur größere Mikrokalzifikate sichtbar).

Um den Einflussgrad der Dichte- und Dickevariationen auf dem Mammogramm zu vermindern, wurde ein Verfahren einer Zwei-Energien-Divisionsmammographie (Verfahren für Differenzdiagnose der Milchdrüse, Patent RU 2391909, 2008) vorgeschlagen.

Dieses Verfahren ist besonders ausführlich in folgenden Druckschriften beschrieben:

1. V.A. Gorshkov, N.I. Rozhkova, S.P. Prokopenko. Zwei- Energien- Divisionsmammographie. Verfahren zur nichtlinearen Analyse für Kardiologie und Onkologie. Physikalische Ansätze und klinische Praxis. Ausgabe 2. OOO KDU Verlag, 2010. S. 173-191.

2. V. Gorshkov, N. Rozhkova, S. Prokopenko. Dual-energy dividing mammography. International Workshop on Digital Mammography. 2010. Girona, Spain. PP.606-613.

Bei der Zwei-Energien-Divisionsmammographie wird das Verhältnis der oben erwähnten Logarithmen visualisiert:

$$\beta = \frac{\mu^L}{\mu^H} = \frac{\ln \frac{N_0^L}{N^L}}{\ln \frac{N_0^H}{N^H}} = k_\beta Z + a_\beta$$

$$(3)$$

Daraus ist es ersichtlich, dass dieses Verhältnis von der Dichte unabhängig ist. Es wird nur durch die Verteilung der effektiven Atomnummern bestimmt.

Fig. 1c zeigt ein Beispiel für das Divisionsmammogramm der gleichen Milchdrüse.

Die Dichte- und Dickevariationen zeigen sich auf dem Divisionsmammogramm weniger ausgeprägt als auf dem Differenzmammogramm. Die Brustwarze der Milchdrüse ist auf dem Differenzmammogramm praktisch nicht sichtbar (sie hat eine sehr kleine Dicke und wird deswegen auch auf dem Divisionsmammogramm praktisch mit gleicher Lichtdichte wie die Milchdrüse dargestellt).

Jedoch zeigt das Mammogramm Gefäße und Gänge und sonstige Dichtevariationen. Das hängt damit zusammen, dass die Gleichung (3) nur für ein Strahlungsspektrum mit einheitlicher Energie zutrifft. Bei realen Spektren der Röntgenröhre, welche sowohl Kenn- als auch Bremsstrahlungen beinhalten, ist es unmöglich, die Dichte- und Dickevariationen in den Mammogrammen zu unterdrücken. Eine wirksame Diagnose der Milchdrüseerkrankungen setzt eine Visualisierung der effektiven Atomnummer, der Dichte und deren Konvexkombination (convex combination) voraus.

Um die Mammogramme mit folgenden dargestellten Verteilungen:

- eine effektive Atomnummer, die gegenüber der Dichtevariation invariant ist,
- eine Dichte der effektiven Atomnummer, die gegenüber der Variation invariant ist,
- die Konvexkombination der effektiven Atomnummer und der Dichte,

zu bekommen, wird ein Verfahren zur Divisions- und Differenz-Mammographie vorgeschlagen, welches auch der Gegenstand der vermutlichen Erfindung ist.

Es ist Aufgabe ein Verfahren zur Divisions- und Differenz-Mammographie zu entwickeln, mit dem auch die Dichtevariation gegenüber invarianten Mammographien erhalten wird.

Die gestellte Aufgabe wird durch die Merkmale des Anspruchs 1 gelöst.

Daraus folgt:

Bei den kontinuierlichen Spektren hängen die zahlenmäßigen Werte — sowohl der Differenzen als auch die Verhältnisse der oben genannten Logarithmen — linear sowohl mit der Dichte (bei konstanter Dicke) als auch mit der effektiven Atomnummer (innerhalb ihres engen Änderungsbereiches) zusammen. Folglich ist die Visualisierung der Verteilung der effektiven Atomnummer und der Dichte aufgrund folgender Gleichungen möglich:

$$Z = k_\alpha \alpha + k_\beta \beta + k_0 = k_\alpha \left( \ln \frac{N_0^L}{N^L} - \ln \frac{N_0^H}{N^H} \right) + k_\beta \frac{\ln \frac{N_0^L}{N^L}}{\ln \frac{N_0^H}{N^H}} + k_0 \quad ,$$

$$\rho = k_\alpha^\rho \alpha + k_\beta^\rho \beta + k_0^\rho = k_\alpha^\rho \left( \ln \frac{N_0^L}{N^L} - \ln \frac{N_0^H}{N^H} \right) + k_\beta^\rho \frac{\ln \frac{N_0^L}{N^L}}{\ln \frac{N_0^H}{N^H}} + k_0^\rho \quad (4)$$

Die Verteilung der konvexen Kombination ihrer Einheitsgrößen wird festgelegt als

$$\lambda = k Z_n + (1 - k)\rho_n$$

wobei k der Koeffizient ($0 \leq k \leq 1$) ist.

$$Z_n = \frac{(Z - Z_{\min})}{(Z_{\max} - Z_{\min})},$$

$$\rho_n = \frac{(\rho - \rho_{\min})}{(\rho_{\max} - \rho_{\min})}$$

Das Problem besteht bei der Bestimmung der Koeffizienten $k_\alpha{}^z$, $k_\beta{}^z$, $k_0{}^z$, $k_\alpha{}^\rho$, $k_\beta{}^\rho$, $k_0{}^\rho$. Um diese Koeffizienten einzuschätzen, wird neben der Mammographie ein Vergleichsmuster mit bekannten Verteilungen für die Dichte, die Dicke und die effektive Atomnummer verwendet (ihre Kennwerte sind der Kennlinie der Milchdrüse ähnlich). Diese Koeffizienten werden anhand der Mammogramme des Vergleichsmusters mit zwei Energien festgestellt.

Dieser Effekt sowie die zahlenmäßige Wiederherstellung der Verteilung der effektiven Atomnummer und der Dichte sind die kennzeichnenden Merkmale des erfindungsgemäßen Verfahrens.

In dieser Beschreibung sind N, $\beta$, $\alpha$, Z, $\rho$, $Z_n$, $\rho_n$, $\lambda$ Matrizen, welche die Werte der entsprechenden Kenngrößen als i,j-ge Pixel eines Detektors (der Mammogramm) festlegen.

Koeffizienten $k_\alpha{}^z$, $k_\beta{}^z$, $k_0{}^z$, $k_\alpha{}^\rho$, $k_\beta{}^\rho$, $k_0{}^\rho$ sind Skalargrößen.

Die Fig. 1d zeigt ein Beispiel für eine Verteilung der effektiven Atomnummer, die mit Hilfe der Zwei- Energien- Divisions- Differenzmammographie anhand von zwei Mammogramme berechnet wurde. Diese zwei Mammogramme wurden bei zwei verschiedenen Plattenspannungen der Röntgenröhre erzeugt. Als Vergleichsmuster wurde ein graphitisches Prisma (simulierendes Gewebe der Milchdrüse) mit Alu- Streifen verschiedener Dicke (simulierende Mikrokalzifikate) verwendet.

Auf dem Divisions-Differenzmammogramm sind praktisch keine Gefäße und Gänge mehr sichtbar. Die gesunde Partie der Milchdrüse wird mit gleicher Lichtdichte dargestellt.

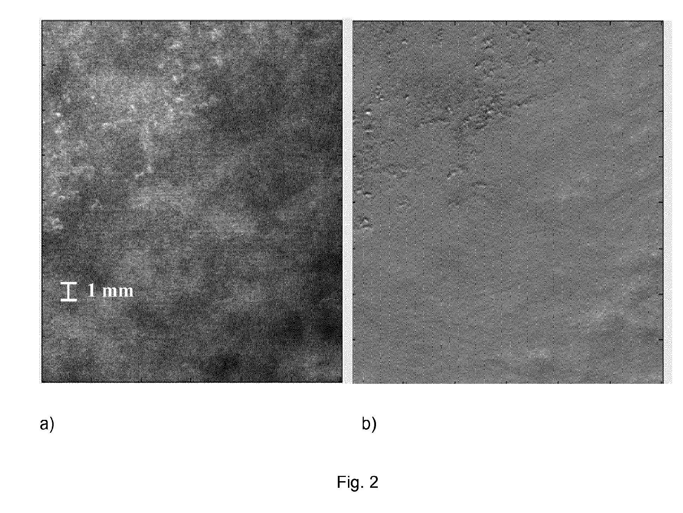

Fig. 2 zeigt Ausschnitte von einem konventionellen und einem Zwei- Energien- Divisions- Differenzmammogramm (Verteilung der effektiven Atomnummer und der Dichte) . Große Mikrokalzifikate lassen sich auf dem konventionellen Mammogramm gut genug erkennen. Die kleinen auf dem Divisions- Differenzmammogramm gut sichtbaren Mikrokalzifikate sind aber nicht sichtbar. Dabei sehen manche Zusammenballungen von kleinen Mikrokalzifikaten aus dem Divisions- Differenzmammogramm auf dem konventionellen Mammogramm wie ein großes Granulum aus.

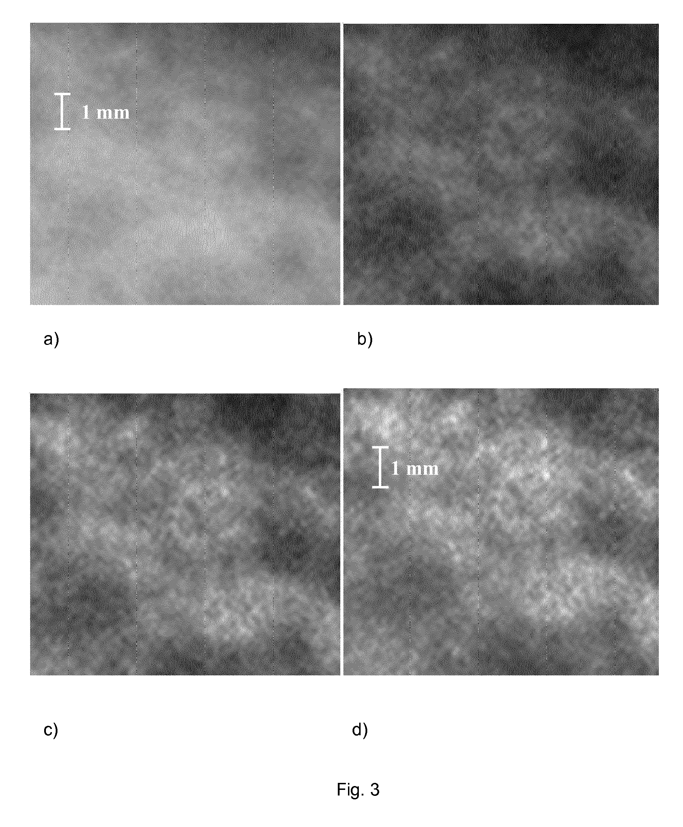

Sowohl ein Krebsgeschwulst als auch die Mikrokalzifikate weisen neben der effektiven Atomnummer auch eine erhöhte Dichte auf. Deswegen lassen sich solche Einschlüsse mittels einer konvexen Kombination ihrer Einheitsgrößen (Normalwerte) besser identifizieren. Fig. 3 schildert die Wirksamkeit der Visualisierung der Konvexkombination der identifizierten Einheitsgrößen der effektiven Atomnummer und der Dichte. Daraus ist ersichtlich, dass die winzigsten Mikrokalzifikate (die bei der konventionellen Screening-Mammographie nicht erkennbar und beim Differenzmammogramm nur schwer erkennnbar sind), hier bei der Verteilung der konvexen Kombination der Einheitsgrößen von effektiver Atomnummer und Dichte sich effektiv genug identifizieren lassen.

Damit ermöglicht die Divisions-Differenzmammographie dass die Erkrankungen der Milchdrüse, die auf die Bildung von Mikrokalzifikaten darin zurückzuführen sind, in einer früheren Phase ihrer Entstehung zu erkennen sind.

Die Erfindung wird anhand der Zeichnungen näher erläutert. Es zeigen:

Fig. 1    Beispiele für Mammogramme

       a — Röntgenscreening-Mammographie,

       b — Zwei- Energien- Differenz- Röntgenmammographie,

       c — das gleiche für eine Divisionsmammographie,

       d — das gleiche für eine Divisions-Differenzmammographie mit der Verteilung der effektiven Atomnummer und

Fig. 2    einen Abschnitt eines konventionellen Mammogramms (a) und eines Divisions-Differenzmammogramms (Verteilung der effektiven Atomnummer) (b).

Fig. 3    Abschnitte der Mammogramme:

a -     bei der Röntgenscreening-Mammographie;

b -     bei der Zwei- Energien- Differenz- Röntgenmammographie;

c -     das gleiche für die Divisionsmammographie;

d -     das gleiche für die Divisions-Differenzmammographie bei einer Verteilung der konvexen Kombination der effektiven Atomnummer und Dichte

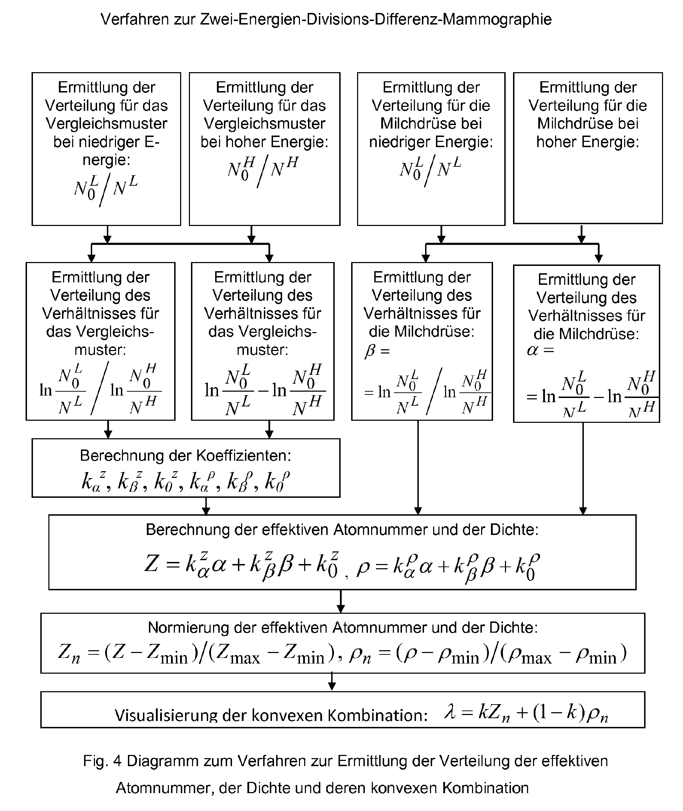

Fig. 4    ein Diagramm zum Verfahren zur Ermittlung der Verteilung der effektiven Atomnummer, der Dichte und deren konvexen Kombination.

Die Erfindung wird in folgenden Schritten ausgeführt:

1. Das Vergleichsmuster mit den bekannten Dichte-, Dicke- und effektiven Atomnummerverteilungen wird neben der Milchdrüse auf der Tischplatte des Mammographen angeordnet.

2. Es werden zwei Mammogramme bei niedriger und hoher Anodenspannung aufgenommen.

3. Die Koeffizienten aus der Formel 4 werden anhand des Vergleichsmusters berechnet.

4. Die Verteilung der effektiven Atomnummern, der Dichte und deren konvexen Kombination in der Brustdrüse wird mit Hilfe der berechneten Koeffizienten visuell dargestellt.

Fig. 4 zeigt das Funktionsprinzip zur Ausführung des Verfahrens zur Zwei- Energien- Divisions- Differenzmammographie.

1. Das Vergleichsmuster mit den bekannten Dichte-, Dicke- und effektiven Atomnummer-Verteilungen wird neben der Milchdrüse auf der Tischplatte des Mammographen angeordnet.

2. Es werden zwei Mammogramme bei niedriger und hoher Anodenspannung aufgenommen. Mit ihrer Hilfe werden die Verteilungen der Verhältnisses der Ausgangsphotonenmenge $N_0$ zur durch den Detektor erfassten Photonenmente sowohl für das Vergleichsmuster als auch für die Milchdrüse bei niedriger (L) und hoher (H) Energie ermittelt.

3. Aufgrund der ermittelten Verteilungen werden die logarithmischen Verteilungen der Verhältnisse und der Differenzen für das Vergleichsmuster und für die Milchdrüse ermittelt:

$$\ln \frac{N_0^L}{N^L} \bigg/ \ln \frac{N_0^H}{N^H} , \quad \ln \frac{N_0^L}{N^L} - \ln \frac{N_0^H}{N^H}$$

4. Aufgrund der Verteilungen der logarithmischen Verhältnisse und Differenzen für das Vergleichsmuster werden die Koeffizienten $k_\alpha^z$, $k_\beta^z$, $k_0^z$, $k_\alpha^\rho$, $k_\beta^\rho$, $k_0^\rho$ des Zusammenhanges der effektiven Atomnummer und der Dichte mit dem Verhältnis und mit diesen Logarithmen in den nachstehenden Gleichungen berechnet

$$Z = k_\alpha^z \left( \ln \frac{N_0^L}{N^L} - \ln \frac{N_0^H}{N^H} \right) + k_\beta^z \frac{\ln \frac{N_0^L}{N^L}}{\ln \frac{N_0^H}{N^H}} + k_0^z \, ,$$

$$\rho = k_\alpha^\rho (\ln \frac{N_0^L}{N^L} - \ln \frac{N_0^H}{N^H}) + k_\beta^\rho \frac{\ln \frac{N_0^L}{N^L}}{\ln \frac{N_0^H}{N^H}} + k_0^\rho$$

5. Anhand der ermittelten Koeffizienten werden die Verteilungen der Verhältnisse ($\beta$) und der Differenz ($\alpha$) der Logarithmen in die Verteilungen der effektiven Atomnummer und der Dichte umgesetzt

$$Z = k_\alpha^z \alpha + k_\beta^z \beta + k_0^z$$

$$\rho = k_\alpha^\rho \alpha + k_\beta^\rho \beta + k_0^\rho$$

Diese Verhältnisse werden für die Diagnose visualisiert.

6. Anhand der Verteilungen der effektiven Atomnummer und der Dichte wird ihre konvexe Kombination berechnet und visualisiert:

$$\lambda = k Z_n + (1-k) \rho_n$$

**Anhang**

Bibliographische Angaben

1. Antoniassi M., Conceição A.L.C. Study of effective atomic number of breast tissues determined using the elastic to inelastic scattering ratio // Nuclear Instruments and Methods in Physics Research Section A: Accelerators, Spectrometers, Detectors and Associated Equipment. 2011. V. 652, № 1. P. 739—743.

2. Frank Carroll, M.D. et al., Tomographic Imaging Using Monochromatic X-rays and Mosaic Crystals in the Geometry of Stationary Source, Object and Detector. Department of Radiology and Radiological Sciences, Vanderbilt University Medical Center, Nashville, TN 37232-2675.

3. Johns, Paul C. et al., "Dual-Energy Mammography: Initial Experimental Results", Medical Physics, May/Jun. 1985, pp. 297-304

4. Lewin, J. M., Isaacs, P. K., Vance, V., Larke, F. J.: Dual-energy contrast-enhanced digital subtraction mammography: feasibility. Radiology, Volume 229, Number 1, 261--268, (2003).

5. Lewin J.M. et al. Clinical comparison of full-field digital mammography and screen-film mammography for detection of breast cancer., AJR Am J Roentgenol., 2002 Sep; 179(3): 671-7.

6. Weij., Hadjiiski L.M. et al. Computer-aided detection systems for breast masses: comparison of performances on full-field digital mammograms and digitized screen-film mammograms., Acad Radiol., 2007 Jun; 14(6): 659-69.

7. Dual energy x-ray imaging system and method for radiography and mammography.Patent 6683934 (USA), 2004.

8. Verfahren zur Diagnose von Brustkrebs zur Ermittlung der Verteilung der effektiven Ordnungszahl bezogen. Patent 2391909, 2008.

9. US 6,173,034 B1

10. US 2009/0304253 A1

11. WO01/040754 A3

**Patentansprüche**

1. Verfahren zur Zwei-Energien-Mammographie mit der Erzeugung von einem Mammogramm mit zwei verschiedenen Strahlungsenergien,
   **dadurch gekennzeichnet,**
   **dass** um die Verteilung der effektiven Atomnummern in der Brustdrüse zwecks Identifikation der Mikrokalzifikate in einer früheren Phase ihrer Entstehung zu erkennen, wird ein Vergleichsmuster mit bekannten Dichte-, Dicke- und effektiven Atomnummernverteilungen neben der Milchdrüse angeordnet,
   **dass** anhand des Vergleichsmusters die Parameter des Zusammenhanges zwischen der Atomnummer und der Differenz bzw. dem Verhältnis der Logarithmen der Anzahl von Fotonen, die die Milchdrüse ohne Zusammenwirkung bei zwei unterschiedlichen Strahlungsenergien durchströmen, bestimmt werden, und dass anhand dieses Zusammenhanges die Verteilung der Atomnummern in der Brustdrüse visuell dargestellt wird.

2. Verfahren nach Anspruch 1,
   **dadurch gekennzeichnet,**
   **dass** um die Mikrokalzifikate sicherer zu erkennen, werden die Dichtevariationen unterdrückt und nur die Variationen der effektiven Atomnummer ausgesondert,
   **dass** bei kontinuierlichen Spektren die zahlenmäßigen Werte - sowohl der Differenzen als auch der Verhältnisse der Logarithmen - linear mit dem Produkt der Dichte und der Dicke als auch mit der effektiven Atomnummer zusammenlaufen, wird zur visuellen Darstellung der Verteilung der effektiven Atomnummer die folgende Gleichung verwendet:

$$ Z = k_\alpha \alpha + k_\beta \beta + k_0 = k_\alpha \left( \ln \frac{N_0^L}{N^L} - \ln \frac{N_0^H}{N^H} \right) + k_\beta \frac{\ln \frac{N_0^L}{N^L}}{\ln \frac{N_0^H}{N^H}} + k_0 \qquad , (4) $$

3. Verfahren nach Anspruch 2,
   **dadurch gekennzeichnet,**
   **dass** die Mammographien der Vergleichsmuster mit zwei Energien aufgenommen und daraus die Koeffizienten $k_\alpha$, $k_\beta$, k0 ermittelt werden.

4. Verfahren der Zwei-Energien-Mammographie mit der Erzeugung von einem Mammogramm mit zwei verschiedenen Strahlungsenergien,
   **dadurch gekennzeichnet,**

**dass**, um die Verteilung der effektiven Atomnummern und der Dichte in der Brustdrüse zwecks Identifikation der Mikrokalzifikate in der früheren Phase ihrer Entstehung zu erkennen und eine schärfere Abbildung der onkologischen Neubildung zu bekommen, ein Vergleichsmuster mit bekannten Dichte-, Dicke- und effektiven Atomnummern-Verteilungen neben der Milchdrüse angeordnet wird; dass anhand des Vergleichsmusters die Koeffizienten der Zusammenhänge zwischen der Atomnummer und der Dichte bzw. dem Verhältnis der Logarithmen der Anzahl von Fotonen, die die Milchdrüse ohne Zusammenwirkung bei zwei unterschiedlichen Strahlungsenergien durchströmen, bestimmt werden, und

**dass** anhand dieser Zusammenhänge die Verteilung dieser Kenndaten und deren konvexe Kombination in der Brustdrüse visuell dargestellt wird.

Fig.1

a)

b)

Fig. 2

a)

b)

c)

d)

Fig. 3

Verfahren zur Zwei-Energien-Divisions-Differenz-Mammographie

| Ermittlung der Verteilung für das Vergleichsmuster bei niedriger Energie: $N_0^L \big/ N^L$ | Ermittlung der Verteilung für das Vergleichsmuster bei hoher Energie: $N_0^H \big/ N^H$ | Ermittlung der Verteilung für die Milchdrüse bei niedriger Energie: $N_0^L \big/ N^L$ | Ermittlung der Verteilung für die Milchdrüse bei hoher Energie: |

| Ermittlung der Verteilung des Verhältnisses für das Vergleichsmuster: $\ln\dfrac{N_0^L}{N^L} \Big/ \ln\dfrac{N_0^H}{N^H}$ | Ermittlung der Verteilung des Verhältnisses für das Vergleichsmuster: $\ln\dfrac{N_0^L}{N^L} - \ln\dfrac{N_0^H}{N^H}$ | Ermittlung der Verteilung des Verhältnisses für die Milchdrüse: $\beta = $ $= \ln\dfrac{N_0^L}{N^L} \Big/ \ln\dfrac{N_0^H}{N^H}$ | Ermittlung der Verteilung des Verhältnisses für die Milchdrüse: $\alpha = $ $= \ln\dfrac{N_0^L}{N^L} - \ln\dfrac{N_0^H}{N^H}$ |

Berechnung der Koeffizienten:
$k_\alpha^z, k_\beta^z, k_0^z, k_\alpha^\rho, k_\beta^\rho, k_0^\rho$

Berechnung der effektiven Atomnummer und der Dichte:

$$Z = k_\alpha^z \alpha + k_\beta^z \beta + k_0^z , \quad \rho = k_\alpha^\rho \alpha + k_\beta^\rho \beta + k_0^\rho$$

Normierung der effektiven Atomnummer und der Dichte:

$$Z_n = (Z - Z_{\min}) \big/ (Z_{\max} - Z_{\min}), \quad \rho_n = (\rho - \rho_{\min}) \big/ (\rho_{\max} - \rho_{\min})$$

Visualisierung der konvexen Kombination: $\quad \lambda = kZ_n + (1-k)\rho_n$

Fig. 4 Diagramm zum Verfahren zur Ermittlung der Verteilung der effektiven Atomnummer, der Dichte und deren konvexen Kombination

**Europäisches Patentamt**
**European Patent Office**
**Office européen des brevets**

# EUROPÄISCHER RECHERCHENBERICHT

Nummer der Anmeldung

EP 12 17 8674

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | VJACHESLAV GORSHKOV ET AL: "Dual-Energy Dividing Mammography", 16. Juni 2010 (2010-06-16), DIGITAL MAMMOGRAPHY : 10TH INTERNATIONAL WORKSHOP, IWDM 2010, GIRONA, SPAIN, JUNE 16-18, 2010 ; PROCEEDINGS, SPRINGER-VERLAG, BERLIN, PAGE(S) 606 - 613, XP019144408, ISBN: 978-3-642-13665-8 | 1,4 | INV. A61B6 |
| A | * das ganze Dokument * | 2,3 | |
| A | WO 99/45371 A1 (IMAGE ANAYLSIS INC [US]; ARNOLD BEN A [US]) 10. September 1999 (1999-09-10) * Zusammenfassung; Anspruch 1; Abbildung 6 * | 1-4 | |
| A | HEISMANN B J ET AL: "Density and atomic number measurements with spectral x-ray attenuation method", JOURNAL OF APPLIED PHYSICS, AMERICAN INSTITUTE OF PHYSICS. NEW YORK, US, Bd. 94, Nr. 3, 1. August 2003 (2003-08-01) , Seiten 2073-2079, XP012059916, ISSN: 0021-8979, DOI: 10.1063/1.1586963 * das ganze Dokument * | 1-4 | RECHERCHIERTE SACHGEBIETE (IPC)<br><br>A61B |
| A | DE 103 05 105 A1 (SIEMENS AG [DE]) 26. August 2004 (2004-08-26) * das ganze Dokument * | 1-4 | |
| A | WO 03/024331 A2 (SIEMENS AG [DE]; HEISMANN BJOERN [DE]; STIERSTORFER KARL [DE]) 27. März 2003 (2003-03-27) * Zusammenfassung; Abbildungen 3-4 * * Seite 5, Zeile 7 - Seite 8, Zeile 9 * | 1-4 | |

-/--

Der vorliegende Recherchenbericht wurde für alle Patentansprüche erstellt

| Recherchenort | Abschlußdatum der Recherche | Prüfer |
|---|---|---|
| München | 30. Oktober 2012 | Martinez Möller, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................

& : Mitglied der gleichen Patentfamilie, übereinstimmendes Dokument

EPO FORM 1503 03.82 (P04C03)

**EP 2 638 857 A1**

Europäisches
Patentamt

European
Patent Office

Office européen
des brevets

# EUROPÄISCHER RECHERCHENBERICHT

Nummer der Anmeldung

EP 12 17 8674

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 93/14419 A1 (CAMBRIDGE IMAGING LTD [GB]) 22. Juli 1993 (1993-07-22) * Seite 21, Absatz 3 - Seite 23, Absatz 2 * ----- | 1-4 | |
| A | US 2008/273666 A1 (WALTER DEBORAH JOY [US] ET AL) 6. November 2008 (2008-11-06) * Zusammenfassung; Anspruch 1 * ----- | 1-4 | |
| A,D | US 6 683 934 B1 (ZHAO JIANGUO [US] ET AL) 27. Januar 2004 (2004-01-27) * Spalte 3, Zeile 36 - Spalte 9, Zeile 22 * ----- | 1-4 | |
| A | LATCHAW: "Effective atomic number and electron density as measured with a computed tomography scanner: computation and correlation with brain tumor histology", JOURNAL OF COMPUTER ASSISTED TOMOGRAPHY, LIPPINCOTT WILLIAMS AND WILKINS, US, 1. April 1978 (1978-04-01), Seiten 199-208, XP009163939, ISSN: 0363-8715 * Seite 199, Absatz 2 - Seite 200, Absatz 2; Abbildung 1 * ----- | 1-4 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 30. Oktober 2012 | Martinez Möller, A |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 12 17 8674

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-10-2012

| Im Recherchenbericht angeführtes Patentdokument | | Datum der Veröffentlichung | Mitglied(er) der Patentfamilie | | Datum der Veröffentlichung |
|---|---|---|---|---|---|
| WO 9945371 | A1 | 10-09-1999 | AU | 2887499 A | 20-09-1999 |
|  |  |  | EP | 1078252 A1 | 28-02-2001 |
|  |  |  | US | 6320931 B1 | 20-11-2001 |
|  |  |  | WO | 9945371 A1 | 10-09-1999 |
| DE 10305105 | A1 | 26-08-2004 | DE | 10305105 A1 | 26-08-2004 |
|  |  |  | US | 2004218728 A1 | 04-11-2004 |
| WO 03024331 | A2 | 27-03-2003 | CN | 1551745 A | 01-12-2004 |
|  |  |  | DE | 10143131 A1 | 03-04-2003 |
|  |  |  | JP | 4309760 B2 | 05-08-2009 |
|  |  |  | JP | 2005501684 A | 20-01-2005 |
|  |  |  | US | 2004223585 A1 | 11-11-2004 |
|  |  |  | WO | 03024331 A2 | 27-03-2003 |
| WO 9314419 | A1 | 22-07-1993 | AU | 1690292 A | 03-08-1993 |
|  |  |  | DE | 69222435 D1 | 30-10-1997 |
|  |  |  | DE | 69222435 T2 | 29-01-1998 |
|  |  |  | EP | 0621959 A1 | 02-11-1994 |
|  |  |  | JP | 3102698 B2 | 23-10-2000 |
|  |  |  | JP | H07505216 A | 08-06-1995 |
|  |  |  | US | 5524133 A | 04-06-1996 |
|  |  |  | WO | 9314419 A1 | 22-07-1993 |
| US 2008273666 | A1 | 06-11-2008 | KEINE | | |
| US 6683934 | B1 | 27-01-2004 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4541106 A
- US 5150394 A
- US 6683934 B
- RU 2391909

- WO 2391909 A
- US 6173034 B1
- US 20090304253 A1
- WO 01040754 A3

**In der Beschreibung aufgeführte Nicht-Patentliteratur**

- **ANTONIASSI M. ; CONCEIÇÃO A.L.C.** Study of effective atomic number of breast tissues determined using the elastic to inelastic scattering ratio. *Nuclear Instruments and Methods in Physics Research Section A: Accelerators, Spectrometers, Detectors and Associated Equipment,* 2011, vol. 652 (1), 739-743
- **LEWIN, J. M. ; ISAACS, P. K. ; VANCE, V. ; LARKE, F. J.** Dual-energy contrast-enhanced digital subtraction mammography: feasibility. *Radiology,* 2003, vol. 229 (1), 261-268
- Zwei- Energien- Divisionsmammographie. Verfahren zur nichtlinearen Analyse für Kardiologie und Onkologie. **V.A. GORSHKOV ; N.I. ROZHKOVA ; S.P. PROKOPENKO.** Physikalische Ansätze und klinische Praxis. OOO KDU Verlag, 2010, 173-191

- **V. GORSHKOV ; N. ROZHKOVA ; S. PROKOPENKO.** Dual-energy dividing mammography. *International Workshop on Digital Mammography,* 2010, 606-613
- **FRANK CARROLL, M.D. et al.** *Tomographic Imaging Using Monochromatic X-rays and Mosaic Crystals in the Geometry of Stationary Source, Object and Detector,* 37232-2675
- **JOHNS, PAUL C. et al.** Dual-Energy Mammography: Initial Experimental Results. *Medical Physics,* Mai 1985, 297-304
- **LEWIN J.M. et al.** Clinical comparison of full-field digital mammography and screen-film mammography for detection of breast cancer. *AJR Am J Roentgenol.,* September 2002, vol. 179 (3), 671-7
- **WEIJ., HADJIISKI L.M. et al.** Computer-aided detection systems for breast masses: comparison of performances on full-field digital mammograms and digitized screen-film mammograms. *Acad Radiol.,* Juni 2007, vol. 14 (6), 659-69